# EUROPEAN PATENT APPLICATION

(11) **EP 1 172 352 A1**
(43) Date of publication of application: **16.01.2002**
(21) Application number: 00116999.4
(22) Date of filing: 08.08.2000
(51) Int. Cl.: C07C 51/50, C07C 55/14, C07C 55/02, C11D 17/00, C11D 3/20

(54) **Retardation of discolouration of dicarboxylic acids**

(30) Priority: 10.07.2000 EP 00870158
(71) Applicant: THE PROCTER & GAMBLE COMPANY, Cincinnati, Ohio 45202 (US)
(72) Inventor: Vanderveken, Walter F. J., 2610 Wilrijk (BE); Pena-Romero, Angelina, 3080 Tervuren (BE); Temmerman, Eric, 9810 Eke (BE)
(74) Representative: Mather, Peter Geoffrey

(57) **Abstract**

According to the present invention there is provided a process for retarding the discolouration of dicarboxylic acids, said process comprising heating said dicarboxylic acid to a temperature above its melting point and adding at least one metal ion sequesterant to said dicarboxylic acid.

A further aspect of the present invention provides a composition comprising at least one dicarboxylic acid and at least one metal ion sequesterant. A further aspect of the present invention provides for the use of metal ion sequesterants to retard the discolouration of dicarboxylic acids, particularly molten dicarboxylic acids.

## Description

### Technical Field

The present invention relates to the retardation of the discolouration of dicarboxylic acids and, in particular, the retardation of the discolouration of molten dicarboxylic acids.

### Background of the Invention

Dicarboxylic acids are used in a variety of ways and are especially valued in the polymer industry where they are used in the production of various polymers including polyamides and polyurethanes. More recently dicarboxylic acids have been found to be useful in the coating of laundry and dishwashing tablets (see, for example, EP-A-846,754).

Many of the uses of dicarboxylic acids require that they be molten during at least part of the process. For example, when hexamethylenediamine is polymerised with hexandioic acid to produce nylon, the polymerisation reaction is usually carried out at by mixing together molten hexamethylenediamine and molten hexandioic acid in stoicheiometric amounts. There are many documents discussing this type of process including US-A-4,131,712, WO-A-96/16107 and EP-A-765,855. Also, as described in EP-A-846,754, the process for coating laundry and dishwashing tablets is preferably carried out using molten dicarboxylic acid.

However, the use of molten dicarboxylic acid can be problematic because molten dicarboxylic acids have a tendency to discolour. This discolouration can be undesirable for many applications. For example, it is desirable to consumers that laundry tablets have a high degree of whiteness. When coated with a discoloured dicarboxylic acid coating such tablets appear dirty to the consumer and, therefore, not suitable for laundry purposes.

Various attempts have been made to solve this problem. For example, US-A-4,442,260 describes a process for making a highly concentrated solution of nylon salt which, through controlling water content and reaction temperatures, claims to reduce the rate of the undesirable side reactions that form the coloured by-products. WO-A-99/61510 describes an improved polyamidation system for producing a polyamide from molten dicarboxylic acid monomer and molten diamine monomer. In the system of WO-A-99/61510 oxygen is removed from the solid dicarboxylic acid by use of a vacuum pump and nitrogen displacement. Ultrasonic vibration under a nitrogen atmosphere is then applied to the molten dicarboxylic acid to eliminate further oxygen. This is said to allow the acid to melt without thermal degradation or discolouration.

However, the prior art solutions can be complex, expensive and are not always suitable or successful. Therefore, there exists a need for a simple, effective way of retarding the discolouration of dicarboxylic acids.

The Applicant has surprisingly found that the presence of metal ion sequesterants can retard the discolouration of dicarboxylic acids. While not wishing to be bound by theory it is believed that metal ions catalyse the undesirable degradation reactions that are thought to cause discolouration. The sequesterants are believed to inhibit the catalytic activity of the metal ions thereby reducing the discolouring side reactions.

### Summary of the Invention

According to the present invention there is provided a process for retarding the discolouration of dicarboxylic acids, said process comprising heating said dicarboxylic acid to a temperature above its melting point and adding at least one metal ion sequesterant to said dicarboxylic acid.

A further aspect of the present invention provides a composition comprising at least one dicarboxylic acid and at least one metal ion sequesterant. A further aspect of the present invention provides for the use of metal ion sequesterants to retard the discolouration of dicarboxylic acids, particularly molten dicarboxylic acids.

Unless otherwise indicated, all ingredients expressed herein are on a weight percentage of the active ingredient.

As used herein the term "metal ion sequesterant" means a component that is effective in sequestering (chelating) metal cations and thus preventing them from acting as simple hydrated cations.

### Brief Description of the Drawings

Figure 1 shows a process according to the present invention for coating tablets in which the metal ion sequesterant(s) are added to a molten bath of dicarboxylic acid.
Figure 2 shows the results, in terms of discolouration as measured by the Hunter-b value, of tablets coated according to the process of Example 1 and Comparative Example 1.

### Detailed Description of the Invention

According to the present invention there is provided a process for retarding the discolouration of dicarboxylic acids, said process comprising, in any order, the steps of heating said dicarboxylic acid to a temperature above its melting point and adding at least one metal ion sequesterant to said dicarboxylic acid. The present process will be described in more detail below.

A further aspect of the present invention provides a composition comprising molten dicarboxylic acid and at least one metal ion sequesterant.

A further aspect of the present invention provides for the use of at least one metal ion sequesterant to retard the discolouration of dicarboxylic acids, particularly molten dicarboxylic acids. Any suitable metal ion sequesterant may be used herein. Preferably the metal ion sequesterant for use herein has calcium and magnesium chelation capacity, but preferentially show selectivity to binding heavy metal ions such as iron, manganese and copper. More preferably, the metal ion sequesterant show selectivity to binding iron ions.

Suitable heavy metal ion sequestrants for use herein include organic phosphonates, such as the amino alkylene poly (alkylene phosphonates), alkali metal ethane 1-hydroxy disphosphonates and nitrilo trimethylene phosphonates.

Preferred among the above species are diethylene triamine penta (methylene phosphonate), ethylene diamine tri (methylene phosphonate) hexamethylene diamine tetra (methylene phosphonate) and hydroxy-ethylene 1,1 diphosphonate, 1,1 hydroxyethane diphosphonic acid and 1,1 hydroxyethane dimethylene phosphonic acid.

Other suitable heavy metal ion sequesterant for use herein include nitrilotriacetic acid and polyaminocarboxylic acids such as ethylenediaminotetracetic acid, ethylenediamine disuccinic acid, ethylenediamine diglutaric acid, 2-hydroxypropylenediamine disuccinic acid or any salts thereof.

Other suitable heavy metal ion sequestrants for use herein are iminodiacetic acid derivatives such as 2-hydroxyethyl diacetic acid or glyceryl imino diacetic acid, described in EP-A-317,542 and EP-A-399,133. The iminodiacetic acid-N-2-hydroxypropyl sulfonic acid and aspartic acid N-carboxymethyl N-2-hydroxypropyl-3-sulfonic acid sequestrants described in EP-A-516,102 are also suitable herein. The β-alanine-N,N'-diacetic acid, aspartic acid-N,N'-diacetic acid, aspartic acid-N-monoacetic acid and iminodisuccinic acid sequestrants described in EP-A-509,382 are also suitable.

EP-A-476,257 describes suitable amino based sequestrants. EP-A-510,331 describes suitable sequestrants derived from collagen, keratin or casein. EP-A-528,859 describes a suitable alkyl iminodiacetic acid sequesterant. Dipicolinic acid and 2-phosphonobutane-1,2,4-tricarboxylic acid are also suitable. Glycinamide-N,N'-disuccinic acid (GADS), ethylenediamine-N-N'-diglutaric acid (EDDG) and 2-hydroxypropylenediamine-N-N'-disuccinic acid (HPDDS) are also suitable.

Preferred sequesterants are selected from diethylene triamine penta(methyl phosphonic) acid, diethylene triamine pentaacetate, ethylene diamine-N,N-disuccinic acid, hydroyethane diphosphonic acid and mixtures thereof. Especially preferred for use herein is hydroyethane diphosphonic acid.

### Process

The present process comprises, in any order, the steps of heating said dicarboxylic acid to a temperature above its melting point and adding at least one metal ion sequesterant to said dicarboxylic acid. Therefore, one aspect of the present process comprises the steps:
(a) heating a dicarboxylic acid to a temperature above its melting point and subsequently;
(b) adding at least one metal ion sequesterant to said dicarboxylic acid.

A further aspect of the present process comprises the steps:
(a) mixing together a dicarboxylic acid and at least one metal ion sequesterant;
(b) heating the mixture to a temperature above the melting point of said dicarboxylic acid

Preferably the dicarboxylic acid is heated to a temperature at least 5°C above its melting point, more preferably at least 10°C above its melting point. Therefore, a preferred process comprises, in any order, the steps:
(a) heating a dicarboxylic acid to a temperature at least 5°C, preferably at least 10°C, above its melting point;
(b) adding at least one metal ion sequesterant to said dicarboxylic acid.

The metal ion sequesterant may be added to the dicarboxylic acid at any time before of after heating. However, preferably the metal ion sequesterant is added within 10 minutes, more preferably within 5 minutes, even more preferably within 2 minutes of the dicarboxylic acid becoming molten. Therefore, a preferred process comprises the steps:
(a) heating a dicarboxylic acid to a temperature above its melting point;
(b) adding at least one metal ion sequesterant to the dicarboxylic acid within 10 minutes, preferably within 5 minutes, more preferably within 2 minutes of the dicarboxylic acid becoming molten.

In the present process the metal ion sequesterant is preferably added in an amount of at least 0.1g per 1,000g of dicarboxylic acid. More preferably the metal ion sequesterant is added in an amount of at least 0.5g per 1,000g of dicarboxylic acid. Even more preferably the metal ion sequesterant is added in an amount of at least 1g per 1,000g of dicarboxylic acid. It is also preferred that the metal ion sequesterant is added in an amount of less than 50g per 1,000g of dicarboxylic acid. More preferably the metal ion sequesterant is added in an amount of less than 15g per 1,000g of dicarboxylic acid. Even more preferably the metal ion sequesterant is added in an amount of less than 5g per 1,000g of dicarboxylic acid.

In a preferred process, water is added to the dicarboxylic acid/metal ion sequesterant mixture. The water may be added at any time but is preferably added after the dicarboxylic acid is molten. Therefore, a preferred process comprises the steps:
(a) heating a dicarboxylic acid to a temperature above its melting point;
(b) adding at least one metal ion sequesterant; and
(c) adding water to the molten dicarboxylic acid

Preferably the water is added within 10 minutes, more preferably within 5 minutes, even more preferably within 2 minutes of the dicarboxylic acid becoming molten. The water may be added in any manner. However, the water is preferably added as liquid or as steam. The water may be added in a single portion, in aliquots or as a continuous stream. For practical purposes the water is preferably added as a continuous stream. The water is preferably added in an amount of at least 1g per 1,000g of dicarboxylic acid. More preferably the water is added in an amount of at least 2.5g per 1,000g of dicarboxylic acid. Even more preferably the water is added in an amount of at least 5g per 1,000g of dicarboxylic acid. The processes according to the present invention are often carried out at a temperature above the boiling point of water. Therefore, the water added in the present process evaporates. If evaporation of the water occurs, it is necessary to add more water to continue to retard the discolouration of the dicarboxylic acid. Therefore, it is preferred that at least 1g of water per 1,000g of dicarboxylic acid is added per minute. More preferably at least 2.5g of water per 1,000g of dicarboxylic acid is added per minute. Even more preferably at least 5g of water per 1,000g of dicarboxylic acid is added per minute.

Therefore, the preferred process of the present invention comprises the step:
(a) heating a dicarboxylic acid to a temperature at least 5°C, preferably at least 10°C, above its melting point;
(b) adding at least one metal ion sequesterant to the dicarboxylic acid; and
(c) adding water to the molten mixture within 10 minutes, preferably within 5 minutes, more preferably within 2 minutes of the dicarboxylic acid becoming molten.
wherein at least 1g, preferably at least 2.5g, more preferably at least 5g, of water per 1,000g of dicarboxylic acid is added per minute.

Preferably the dicarboxylic acid for the present process is selected from C₂-C₁₃ dicarboxylic acids and mixtures thereof, more preferably C₆-C₁₂ dicarboxylic acids and mixtures thereof. Preferred for use herein are hexanedioic acid, octanedioic acid, decanedioic acid, dodecanedioic acid and mixture thereof. More preferred for use herein is hexanedioic acid.

It is preferred that the compositions of the present process do not contain any clay mineral compound. As used herein the term "clay mineral compound" means hydrous phyllosilicate, typically having a two or three layer crystal structure.

### Composition

The compositions of the present invention comprise molten dicarboxylic acid and at least one metal ion sequesterant. They may comprise other, optional ingredients. However, if present, the compositions herein preferably comprise less than 10%, more preferably less than 5%, by weight, of optional ingredients. The present compositions preferably comprise at least 0.01%, more preferably at least 0.05%, even more preferably at least 0.1% by weight of metal ion sequesterant. It is also preferred that the present compositions comprise less than 5%, more preferably less than 1.5%, even more preferably less than 0.5% by weight of metal ion sequesterant.

### Dicarboxylic Acids

Any dicarboxylic acid or mixture of dicarboxylic acids may be used herein. However, preferably the dicarboxylic acid is selected from C₂-C₁₃ dicarboxylic acids and mixtures thereof, more preferably C₆-C₁₂ dicarboxylic acids and mixtures thereof. Preferred for use herein are hexanedioic acid, decanedioic acid, octanedioic acid, dodecanedioic acid and mixture thereof. More preferred for use herein is hexanedioic acid. Preferably the dicarboxylic acids for use herein have a melting point of from 80°C to 200°C, more preferably from 90°C to 180°C, even more preferably from 95°C to 160°C.

### Metal Ion Sequesterants

Any suitable metal ion sequesterant may be used herein. Preferably the metal ion sequesterant has calcium and magnesium chelation capacity, but preferentially shows selectivity to binding heavy metal ions such as iron, manganese and copper. More preferably, the metal ion sequesterant show selectivity to binding iron ions. Preferably the sequesterant for is selected from phosphonates, carboxylates and mixtures thereof.

Suitable heavy metal ion sequestrants for use herein include organic phosphonates, such as the amino alkylene poly (alkylene phosphonates), alkali metal ethane 1-hydroxy disphosphonates and nitrilo trimethylene phosphonates.

Preferred among the above species are diethylene triamine penta (methylene phosphonate), ethylene diamine tri (methylene phosphonate) hexamethylene diamine tetra (methylene phosphonate) and hydroxy-ethylene 1,1 diphosphonate, 1,1 hydroxyethane diphosphonic acid and 1,1 hydroxyethane dimethylene phosphonic acid.

Other suitable heavy metal ion sequesterant for use herein include nitrilotriacetic acid and polyaminocarboxylic acids such as ethylenediaminotetracetic acid, ethylenediamine disuccinic acid, ethylenediamine diglutaric acid, 2-hydroxypropylenediamine disuccinic acid or any salts thereof.

Other suitable heavy metal ion sequestrants for use herein are iminodiacetic acid derivatives such as 2-hydroxyethyl diacetic acid or glyceryl imino diacetic acid, described in EP-A-317,542 and EP-A-399,133. The iminodiacetic acid-N-2-hydroxypropyl sulfonic acid and aspartic acid N-carboxymethyl N-2-hydroxypropyl-3-sulfonic acid sequestrants described in EP-A-516,102 are also suitable herein. The β-alanine-N,N'-diacetic acid, aspartic acid-N,N'-diacetic acid, aspartic acid-N-monoacetic acid and iminodisuccinic acid sequestrants described in EP-A-509,382 are also suitable.

EP-A-476,257 describes suitable amino based sequestrants. EP-A-510,331 describes suitable sequestrants derived from collagen, keratin or casein. EP-A-528,859 describes a suitable alkyl iminodiacetic acid sequesterant. Dipicolinic acid and 2-phosphonobutane-1,2,4-tricarboxylic acid are also suitable. Glycinamide-N,N'-disuccinic acid (GADS), ethylenediamine-N-N'-diglutaric acid (EDDG) and 2-hydroxypropylenediamine-N-N'-disuccinic acid (HPDDS) are also suitable.

Preferred sequesterants are selected from diethylene triamine penta(methyl phosphonic) acid, diethylene triamine pentaacetate, ethylene diamine-N,N-disuccinic acid, hydroyethane diphosphonic acid and mixtures thereof. Especially preferred for use herein is hydroyethane diphosphonic acid.

It is preferred that the present compositions do not contain any clay mineral compound.

### Preferred Embodiment

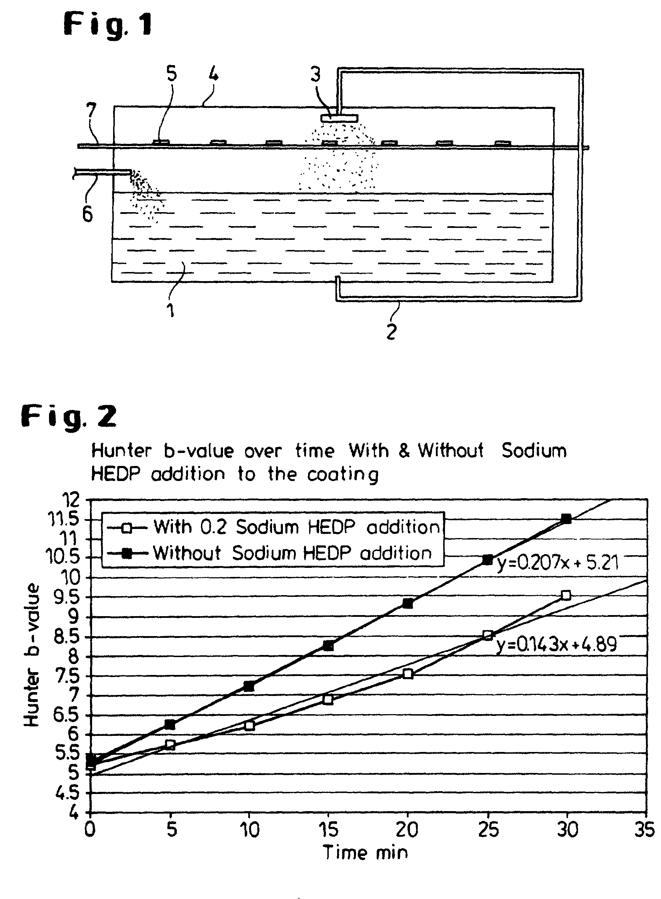

A preferred embodiment of the present invention is shown in Figure 1. In Figure 1, dicarboxylic acid and hydroyethane diphosphonic acid are added to container (4) and then heated with mixing until the dicarboxylic acid is molten. Water is added to the molten dicarboxylic acid (1) through an inlet pipe (6) at a rate of 30g/min. The molten mixture is then pumped through a pipe (2) to a shower head (3). The shower head is positioned over a conveyer belt (7) carrying detergent tablets (5). The molten dicarboxylic acid is then sprayed over the tablets. The tablets are thus coated with a dicarboxylic acid coating. The excess coating is blown off and the tablets are transported to the outside of the box.

### Optional Ingredients

The compositions herein can comprise other optional ingredients. A variety of optional ingredients may be useful. These include ion exchange resins such as PUROLITE™ (available from Purolite International Ltd, Rhondda-Cynon-Taff, UK), integrity improving polymers such as OPADRY™ (available from Colourcon Ltd, Dartford, Kent, UK), -0H donators, heat absorbers such as silicone oil or plasticisers such as terephtalate.

### Examples

The following examples further illustrate the preferred embodiments within the scope of the present invention. The examples are given solely for the purposes of illustration and are not to be construed as limitations of the present invention as many variations of the invention are possible without departing from its spirit or scope.

### Example 1

9500g of hexanedioic acid was mixed with 500g of PUROLITE™ and 20g of HEDP in a container and then heated to a temperature of 165°C with mixing. This molten mixture was pumped through a pipe to a shower head. The shower head was positioned over a conveyer belt carrying detergent tablets. The tablets are thus coated with a hexanedioic acid coating. The excess coating is blown off and the tablets are transported to the outside of the box.

Sample tablets were collected every 5 minutes for a 30 minute period and the level of discoloration, as measured by the Hunter-b value, was assessed. The discolouration was assessed using a Minolta Chroma Meter DP301 with a CR310 Lens. The Chroma Meter was calibrated against a white calibration plate and then the tip of measuring head was placed flat against the centre of the tablet surface. The results are shown in Figure 2.

### Comparative Example 1

Detergent tablets were coated in the exactly the same way as in Example 1 except that no metal ion sequesterant was added to the molten dicarboxylic acid. Again, sample tablets were collected every 5 minutes for a 30 minute period and, using the above described method, the level of discoloration, as measured by the Hunter-b value, was assessed. The results are shown in Figure 2.

| | **Example 3 (wt %)** | **Example 4 (wt %)** | **Example 5 (wt %)** | **Example 6 (wt %)** |
|---|---|---|---|---|
| Hexanedioic Acid | 94.7 | 94.7 | 94.4 | - |
| Dodecanedioic acid | - | - | - | 94.6 |
| Water | 0.2 | 0.2 | 0.5 | 0.3 |
| HEDP¹ | - | 0.1 | 0.1 | 0.1 |
| DTPA² | 0.1 | | | |
| Purolite³ | 5 | 5 | 5 | 5 |

| | | | | |
|---|---|---|---|---|
| ¹hydroyethane diphosphonic acid | | | | |
| ²diethylene triamine penta acetate | | | | |
| ³available from Purolite International ltd, Rhondda-Cynon-Taff, UK | | | | |

## Claims

1. A process for treating dicarboxylic acids, said process comprising, in any order, the steps:
(a) heating said dicarboxylic acid to a temperature above its melting point;
(b) adding at least one metal ion sequesterant to said dicarboxylic acid.

2. A process according to Claim 1 wherein the metal ion sequesterant is selected from those which are able to form complexes with iron ions.

3. A process according to Claim 1 or 2 wherein the dicarboxylic acid is selected from C₂-C₁₃ dicarboxylic acids and mixtures thereof, preferably C₆-C₁₂ dicarboxylic acids and mixtures thereof, more preferably hexanedioic acid.

4. A process according to any of the preceding claims at least 0.1g, preferably at least 1g of metal ion sequesterant is added per 1,000g of dicarboxylic acid.

5. Use of metal ion sequesterants to retard the discolouration of molten dicarboxylic acids.

6. A use according to Claim 7 wherein the dicarboxylic acid is selected from C₂-C₁₃ dicarboxylic acids and mixtures thereof, preferably C₆-C₁₂ dicarboxylic acids and mixtures thereof, more preferably hexanedioic acid.

7. A composition comprising molten dicarboxylic acid and at least one metal ion sequesterant.

8. A composition according to Claim 8 comprising at least 0.01%, more preferably at least 0.05%, even more preferably at least 0.1%, by weight, of metal ion sequesterant

9. A composition according to Claim 9 wherein the dicarboxylic acid is selected from C₂-C₁₃ dicarboxylic acids and mixtures thereof, preferably C₆-C₁₂ dicarboxylic acids and mixtures thereof, more preferably hexanedioic acid.

10. A process for coating detergent tablets with a dicarboxylic acid, wherein said dicarboxylic acid is retarding from discolouring, said process comprising:
(a) heating a dicarboxylic acid to a temperature above its melting point;
(b) adding at least one metal ion sequesterant to said dicarboxylic acid;
(c) applying the dicarboxylic acid to the tablet.
